# EUROPEAN PATENT APPLICATION

(11) **EP 4 035 702 A1**
(43) Date of publication of application: **03.08.2022**
(21) Application number: 21154255.0
(22) Date of filing: 29.01.2021
(51) Int. Cl.: A61M 1/14

(54) **A DIALYSIS MACHINE AND CONTROL METHOD FOR A DIALYSIS MACHINE**

(71) Applicant: Bellco S.r.l., 41037 Mirandola (IT)
(72) Inventor: MARRA, Antonio Giuseppe, 41037 Modena (IT)
(74) Representative: Maschio, Antonio

(57) **Abstract**

A dialysis machine comprising a battery for, in use, powering the machine. A battery management system is provided for monitoring the battery and determining therefrom a battery condition. A controller is provided for determining whether the battery condition is sufficient for performing a dialysis procedure on a patient and in the event of determining that the battery condition is not sufficient, a remedial state in entered. In the remedial state the battery may be replaced or recharged to a level sufficient for the battery condition to be sufficient for carrying out the procedure. A method is also disclosed.

## Description

### FIELD OF THE INVENTION

This invention relates to a dialysis machine and a control method for such a machine.

### BACKGROUND

One type of dialysis for the treatment of kidney failure is peritoneal dialysis (PD) which uses the lining of a patient's abdomen, the peritoneum, to filter blood while it remains inside the body. Peritoneal Dialysis is an effective way to treat patients with End Stage Renal Disease and acute patients with renal failure. The patient is provided with a catheter that includes a transfer set for selective connection to a dialysis solution or a drain bag. A dialysis solution ('dialysate' or 'peritoneal dialysis fluid') comprising water with other electrolytes is warmed to body temperature and then introduced into the peritoneal cavity of the patient via the catheter. The solution remains here for a period of time required to clean the blood of waste products, toxins and excess fluid and these are then removed from the body to leave the right amounts of electrolytes and nutrients in the blood. The machine or 'cycler' may repeat the exchange process a number of times (cycles), as required for a particular patient.

Conventionally dialysate consists of purified water, glucose and electrolytes, with the concentration of electrolytes resembling that which occurs naturally in the blood. It is prepared according to an individual patient's needs to help regulate their electrolyte and acid-base balance and remove metabolic waste products. The dialysate components are normally shipped in the form of concentrates, with basic and acidic concentrates being prepared separately. The basic concentrate is usually supplied as a dry concentrate consisting of pure sodium bicarbonate which is made up into a saturated solution and the acidic concentrate is usually provided as a liquid concentrate. If it is provided as a dry concentrate, the entire concentrate must be dissolved before being diluted to form the dialysate. This requires a large volume of liquid and multiple components for providing the peritoneal dialysis fluid, increasing the time required for production of the fluid. Concentrate may also be wasted during the production of the correct peritoneal dialysis fluid and difficulties may be encountered in providing the correct concentration of electrolytes in the fluid for a particular patient.

In a newly developed dialysis machine, it has been proposed to provide an architecture in which one part of the machine will be used to prepare the dialysate from purified water (purifying a domestic water supply) and the concentrate and another part to cycle the prepared dialysate through the patient via a catheter into the patent's peritoneum. That part of the machine where the dialysate is prepared is called a preparator.

It will be understood that dialysis machines require a source of electrical power to operate. It will be further understood that the source of that power must be reliable in order to ensure that the dialysis may be reliably performed. Even in developed countries, power outages or cuts to electrical power supply can regularly occur. Whilst it is not unusual for professional healthcare environments such as clinics and hospitals to be equipped with back-up power generation to mitigate the effects of such power outages, domestic power supplies rarely have such power backup. In developing countries power outages are a regular occurrence.

To mitigate these problems, it has been proposed to provide a dialysis machine or apparatus which includes a battery power supply. However, the provision of such a battery power supply itself may cause further problems. A battery power supply will have a finite capacity which will require replacement of the battery power supply or recharging where it is a rechargeable type. Rechargeable batteries have a lifetime expressed in terms of duration of use or number of charging cycles carried out. Over that lifetime, the capacity of the battery will fall. Initially, the reduction in capacity is small but the drop -off becomes more rapid as the lifetime recharging cycles is approached. Further, manufacturing defects or use outside of the designed operating parameters of the battery may lead to more rapid "aging". For critical medical equipment, such as dialysis machines it is important that a power supply has predictable performance in order to avoid failure during what may be a significant medical treatments.

The present invention arose in an attempt to at least mitigate some of these problems.

### SUMMARY OF THE INVENTION

According to the invention there is provided a dialysis machine comprising a battery for, in use, at least in part powering the machine, a battery management system for monitoring the battery and determining therefrom a battery condition, a controller for determining whether the battery condition is sufficient for performing a dialysis procedure on a patient and in the event of determining that the battery condition is not sufficient entering a remedial state.

By determining that the battery condition is sufficient for the proposed procedure, the procedure may be safely started without the possibility of the battery supply failing during the procedure. The procedure may be the whole dialysis operation performed on the patient or part of it.

By battery condition, it is meant parameters such as voltage, charge state or the ability to support a certain current draw.

In the preferred embodiment, the battery is a rechargeable battery and the apparatus includes a charging circuit. In embodiments, the battery may be made removeable to allow it to be replaced or recharged out of the apparatus. Various battery technologies may be used such as dry cell, nickel cadmium, lithium metal hydride or lithium-ion polymer.

Whilst in some embodiments, the battery alone will be used to power the dialysis machine, it is preferable that the machine comprises a mains-powered power supply providing a power supply to the dialysis machine and comprising a mains supply power failure detector which in the event of the determination of a mains power supply failure couples the battery to power at least some of the dialysis machine functions. In this preferred way the battery is a back-up battery to cater for loss of a mains power supply.

Preferably the apparatus comprises a memory for, in use, storing battery conditions, a battery state detector for comparing a measured parameter or parameters of the battery and the stored battery conditions to determine a battery condition. The memory may be provided as ROM or RAM. The data held may be preloaded to the memory during manufacture or my adding later. The data may be revised by downloading or upon installation of a new battery. The data may also be provided from the battery itself by the use of so-called "smart" batteries.

Advantageously, the means to apply to the battery a load such that the measured battery parameter is a parameter for the battery operating under load conditions.

Preferably, a battery sensor is provided for determining one or more of the parameters of: voltage, current and temperature.

Preferably, the detected voltage is compared with a voltage threshold and the dialysis machine is placed in the remedial state if the threshold is not exceeded.

Preferably, in the remedial state performance of the dialysis procedure is at least in part prevented. Certain parts of the dialysis procedure may be considered to be critical for patient safety and should only be fully carried out once started or the whole dialysis procedure may need to be carried out.

On entering the remedial state, visual or audible alarms may be provided to prompt remedial action. These may be given by a user interface such as led, Icd lights or displays, bells and sounding devices.

Preferably, when in the remedial state the battery is coupled to a battery charger to charge the battery.

According to a second aspect of the invention there is provided a method of battery management in a dialysis machine comprising; monitoring the battery to determine a battery condition, comparing the determined battery condition with predetermined battery characteristics to determine if the battery has sufficient capacity to enable the dialysis machine to complete at least in part a dialysis procedure on a patient and, in the event that the battery capacity is insufficient, to complete the dialysis procedure entering a remedial state.

Preferably, in the remedial state at least one of the following steps are carried out; replacement of the battery, recharging of the battery, indicating a fault condition.

Preferably, the method includes a step of loading a memory with battery characteristics against which determined battery condition is compared with the battery condition to determine a corresponding present capacity of the battery.

The method of battery management may be held in memory as a set of instructions to be performed by a processor.

### BRIEF DESCRIPTION OF THE FIGURES

A specific embodiment of the invention will now be described with reference to the figures in which:
Figure 1 shows in schematic form a dialysis machine in accordance with an embodiment of the invention in which a preparator module of the machine is located in a patient's bathroom and a cycler module of the machine is located in the patient's bedroom.
Figure 2 is a block diagram of the cycler shown in figure 1 and a power management system; and
Figure 3 shows schematically a battery test operation used in the power management system of figure 2.

### DETAILED DESCRIPTION

### Definitions

Unless defined otherwise, all technical and scientific terms used generally have the same meaning as commonly understood by one of ordinary skill in the art.

The articles "a" and "an" are used to refer to one or to over one (i.e., to at least one) of the grammatical object of the article. For example, "an element" means one element or over one element.

The term "comprising" includes, but is not limited to, whatever follows the word "comprising." Use of the term indicates the listed elements are required or mandatory but that other elements are optional and may be present.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The term "dialysate" describes a fluid into or out of which solutes from a fluid to be dialyzed diffuse through a membrane. An initial dialysate used for therapy typically contains electrolytes close in concentration to the physiological concentration of electrolytes found in blood. However, the concentration of the dialysate can change over the course of therapy, and can further be adjusted as desired.

The term "dialysis flow path" refers to a fluid pathway or passageway configured to convey a fluid, such as dialysate and/or blood, wherein said pathway forms at least part of, preferably the whole of, a fluid circuit for peritoneal dialysis, haemodialysis, hemofiltration, hemodiafiltration or ultrafiltration. A dialysis machine may be included within the flow path. A dialyzer may be included in the flow path.

The term "mix" means to combine one or more substance so that the resulting mixture is not easily separated. Mixing, especially evenly spreading, of solute and solvent aids and speeds the process of dissolution. Mixing can be achieved by any method, including spraying, stirring, shaking or otherwise agitating. The term "improved mixing" refers to a situation wherein the components of a mixture are more evenly distributed and not clumped together. Improved mixing may be achieved, for example, by speeding the mixing up. In the context of a solution, improved mixing results in a solution of the correct concentration because all or most of the solutes dissolve, rather than remaining as clumps or aggregated within the solvent.

The present invention relates to an improved automated peritoneal dialysis machine that includes a preparator and cycler for in situ preparation of peritoneal dialysis fluid (PDF) for delivery and drainage from a patient with communications links established therebetween.

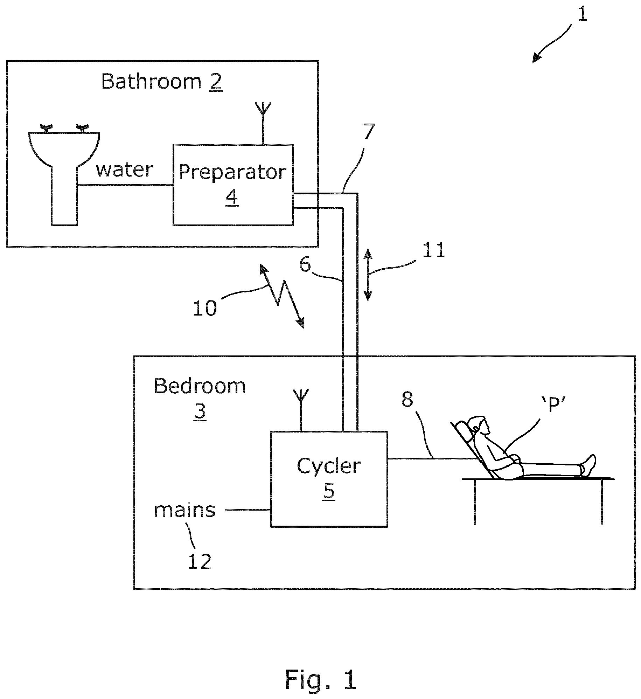

Fig. 1 shows an example of a peritoneal dialysis machine 1 according to the present invention. The peritoneal dialysis machine 1 is set up in the patient's home which is represented by two rooms, a bathroom 2 and an adjoining bedroom 3 (although these rooms are representative only and the locations could be other rooms). The peritoneal dialysis machine 1 includes a preparator 4, an automatic cycler 5, a flexible dialysate supply tube 6 and an electrical power supply line 7.

The preparator 4 is positioned in bathroom 2 as it requires a water source to enable the preparator 4 to generate peritoneal dialysis fluid. The water source may comprise, for example, a tap (faucet) or a more permanent outlet. The preparator 4 first filters the input water and mixes it with a quantity of powered dialysate in accordance with a preprogramed formulation. The powered dialysate will include dextrose, magnesium and calcium and lactate bicarbonate and sodium chloride

The automatic cycler 5 is positioned in the bedroom 3 where the patient "P" intends to undergo peritoneal dialysis. The cycler 5 is connected to a dialysate delivery tube 8 which is connected to a catheter (not shown) which is inserted into the peritoneum of the patient. The cycler 5 controls the flow of the dialysate into, and out of, the patient in accordance with the required dialysis procedure. Dialysate removed from the patient is stored in a container in the cycler 5 (not shown) for subsequent disposal. This procedure is predetermined and held in memory within the cycler 5 operated under processor control. Broadly, the machine operates by preparing dialysate at the preparator 4, the prepared dialysate is pumped via the dialysate supply tube 6 to the cycler 5. There it is heated to patient temperature and administered to the patent via the delivery tube 8 and the catheter into the patient. After, the appropriate time-period for dialysis, the dialysate is removed from the patient via catheter and the delivery tube 8 and held in storage for subsequent removal and disposal.

As will be appreciated, although the two modules are not collocated, it is necessary for instructions and data to pass between the modules. For example, the preparator 4 will need to provide status updates to the cycler 5 indicating that the dialysate is mixed and available for delivery. The cycler 5 will need to instruct the preparator 4 to commence with delivery, or to increase, or reduce the rate of delivery. Error states may need to be passed. For example, the preparator 4 may experience a problem with the water supply pressure and that will need to be communicated to the cycler and indicated as a fault condition on a display. Accordingly, the preparator 4 and the cycler 5 are each provided with a suitably programmed processor to control its respective functions. In addition, a master processor or controller is provided or designated to control the function of the machine as a whole. These processors (or one processor programmed to provide a number of processing functions) provide in addition a communication function to allow the modules to intemperate and to be controlled.

In this embodiment of the invention, there are two communications links which are operable to enable communications between the modules. A first communications link 10 using radio and operating in accordance with a Wi Fi communications protocol and a physical communications link 11 provided by cable 7 over which a Powerlink communications protocol is used.

In order to move the dialysate from the preparator 4 to the cycler 5 and to and from the patient, a pump or pumps are required. In this case the pump is located at the cycler 5 but it could be located at the preparator or indeed two or more pumps could be provided located at each module.

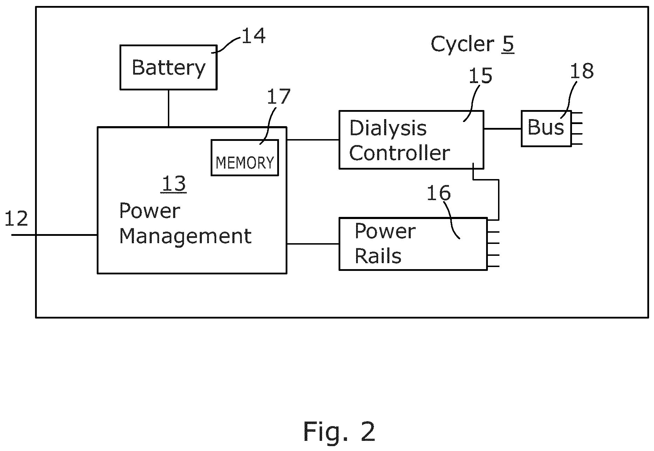

It will be seen that the cycler 5 is connected to a domestic mains electrical power supply 12. This provides the electrical power for both the cycler and also the preparator 4 via a power cable 7 from the cycler. As is shown in figure 2, the cycler includes a power management system 13 formed by a suitably programmed microprocessor with associated memory.

The power management system 13 is also connected to a battery 14. In this case, it is a 12v DC lead acid battery with a nominal capacity of 10aH (amp hours) although other battery types may be used such as based on nickel cadmium, lithium-ion polymer technologies etc offering different nominal voltages and capacities. More than one battery may be provided to reach the desired capacity or voltage. For example, 24Vdc may be provided from series connection of two 12V batteries.

It will be appreciated that the domestic mains power supply 12 will be at a high AC voltage. This will vary from country to country although most countries have a supply in the range 220 to 240 volts at 50 to 60Hz. (The USA is a notable exception of 120 V at 60Hz.) Accordingly, the power management system 13 includes a transformer to convert the supplied voltage to a DC voltage at level suitable for charging the battery 14. For example, a typical charging voltage for a 12v battery may be 13.8 v or a slightly higher voltage may be applied for short periods. Other types of batteries will require particular charging profiles. The charging process is managed by the power management system 13 connecting the battery to a suitable charging circuit.

The battery 14 provides a power supply in the event of a power outage being detected under the control of the power management system 14. The power is provided to power rails 16 which provide power to all parts of the dialysis machine. In normal operation when there is not a power outage, the power management system 13 provides voltage derived and rectified from the mains power supply. The power management system also includes a memory 17 to hold data about the battery 14. This may be data entered by the user or data downloaded from the battery itself.

The operation of the dialysis is controlled by a dialysis controller 15. This provides control commands via a data and control bus 18 to all parts of the dialysis machine including the preparator section 4 and associated pumps, mixers and heaters.

Before any dialysis is carried out, a test procedure or method is performed. In this, the battery 14 is tested under load to ensure that the battery is able to provide adequate power for the anticipated duration of a dialysis. The load may be provided as a set of resistances to mimic the load of the rest of the dialysis machine under operative conditions or may involve activation of the elements of the machine as if dialysis was taking place by for example operating the pumps and heaters and or other parts of the dialysis machine collectively or independently.

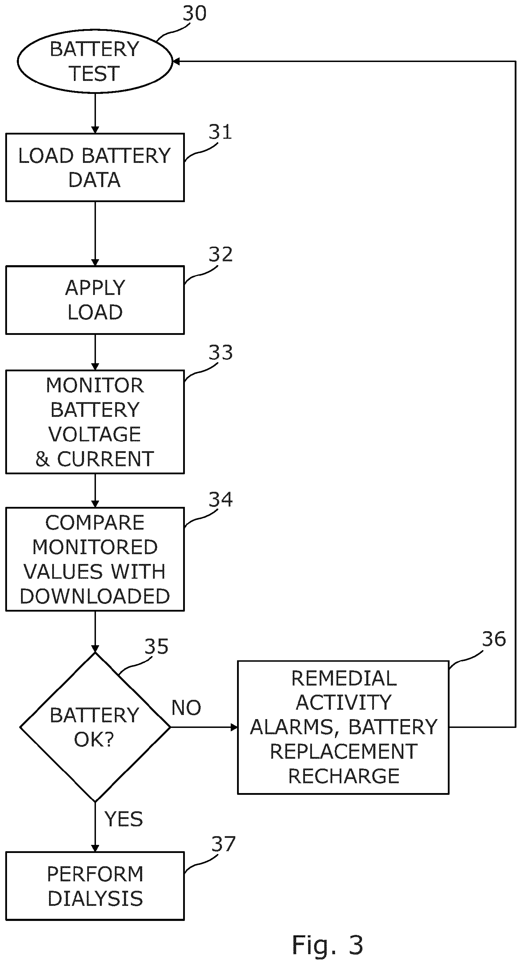

The test procedure 30 will now be described with reference to figure 3. In a first step 31, battery data is loaded into memory 17. This will include battery type, capacity, and voltage. For different battery types there may be provided data concerning the capacity profile with age of the battery. In this case the battery is lead acid battery, 12 volts with a capacity of 10Ah (amp hours). Further, a voltage figure when under the load of dialysis machine is provided.

In step 32, a load is applied to the battery 14. This may be as the result of the activation of the pump and heater of the dialysis machine or by connection of a circuit including resisters to mimic the load under the control of the power management system 13 by, for example, the power management system 13 instructing the dialysis controller 15 to perform a test operation in which these components are instructed to operate.

In step 33, the battery voltage and the current drawn are monitored by the power management system 13. The power management system 13 compares the monitored values with the battery data held in memory 17 in step 34.

In step 35 a determination is made as to whether or not the battery has sufficient remaining capacity to perform a dialysis operation. This may be done by reference to power curve data for the battery or simply on the basis of the monitored voltage level under load conditions being above a predetermined voltage threshold. For example, a battery voltage may vary under test/ load as follows.

| Charge % | Voltage under load |
|---|---|
| 100 | 12.73 |
| 90 | 12.62 |
| 80 | 12.50 |
| 70 | 12.37 |
| 60 | 12.24 |
| 50 | 12.10 |
| 40 | 11.96 |
| 30 | 11.81 |
| 20 | 11.66 |
| 10 | 11.51 |

For a dialysis procedure to be safely performed, it may require that the battery has at least 60 % of its capacity. Hence the voltage threshold will be set to be 12.24 V. Anything under that threshold will result in a branch to a remedial activity procedure 36. This will include waiting for a charging operation to complete, replacement of the battery 14 or other servicing or alarm procedures to rectify any issue.

Following those remedial activities, the process returns to the battery test procedure 30.

If the voltage is above the threshold, then the dialysis may be carried out in step 37.

It will be appreciated that other ways of determining the remaining battery capacity may be utilised.

For example, state of charge curves may be held in memory and compared with those generated by testing the battery under load. It may also be possible to determine an off-load voltage although a voltage under load conditions is preferred as this will give a more predictable indication of available remaining battery capacity.

The measure voltage may also be used to indicate a battery fault. For example, if zero volts is detected then the battery may be missing, not connected or damaged, and a fault indicated to a user interface.

The user interface may include a led or liquid crystal display or lamps or lights for example.

Whilst in the described example, the battery is provided as a back-up power source in the event of a power failure, in some embodiments the battery may be used as a power source for the dialysis in a primary sense or to provide the power source for part of the dialysis operation. For example, the preparator may require to mix for a number of hours and the power for this may be provided by the battery before mains power is selected to provide the power to pump and heat etc during the next phase of the dialysis. The battery may also be the sole power source for the dialysis machine with battery power management system determining that the battery must be replaced before dialysis can take place.

One skilled in the art will understand that various combinations and/or modifications and variations can be made in the described apparatus, methods and uses depending upon the specific needs for operation. Moreover, features illustrated or described as being part of an aspect of the invention may be used in the aspect of the invention, either alone or in combination.

## Claims

1. A dialysis machine comprising a battery for in use powering the machine, a battery management system for monitoring the battery and determining therefrom a battery condition, a controller for determining whether the battery condition is sufficient for performing a dialysis procedure on a patient and in the event of determining that the battery condition is not sufficient entering a remedial state.

2. A dialysis machine as claimed in claim 1 comprising a mains-powered power supply providing a power supply to the dialysis machine and comprising a mains supply power failure detector which in the event of the determination of a mains power supply failure couples the battery to power at least some of the dialysis machine functions.

3. A dialysis machine as claimed in claim 1 or 2 wherein the battery is rechargeable battery.

4. A dialysis machine as claimed in claim 3 further comprising a battery charging circuit for charging the battery.

5. A dialysis machine as claimed in any preceding claims comprising memory for, in use, storing battery conditions, a battery state detector for comparing a measured parameter or parameters of the battery and the stored battery conditions to determine a battery condition.

6. A dialysis machine as claimed in claim 5 comprising means to apply to the battery a load such that the measured battery parameter is a parameter for the battery operating under load conditions.

7. A dialysis machine as claimed in any preceding claim wherein a battery sensor is provided for determining one or more of the parameters of: voltage, current and temperature.

8. A dialysis machine as claimed in claim 7 wherein the detected voltage is compared with a voltage threshold and the dialysis machine is placed in the remedial estate if the threshold is not exceeded.

9. A dialysis machine as claimed in any preceding claim wherein in the remedial state performance of the dialysis procedure is at least in part prevented.

10. A dialysis machine as claimed in any preceding claims wherein an alarm is provided in the remedial state.

11. A dialysis machine as claimed in any preceding claims wherein when in the remedial state the battery is coupled to a battery charger to charge the battery.

12. A method of battery management in a dialysis machine comprising:
monitoring the battery to determine a battery condition;
comparing the determined battery condition with predetermined battery characteristics to determine if the battery has sufficient capacity to enable the dialysis machine to complete a dialysis procedure on a patient and, in the event that the battery capacity is insufficient, to complete the dialysis procedure entering a remedial state.

13. A method as claimed in claim 12 wherein in the remedial state at least one of the following steps are carried out: replacement of the battery; recharging of the battery, indicating a fault condition.

14. A method as claimed in claim 12 or 13 comprising the step of loading a memory with battery characteristics which are compared with the determined battery condition to determine a corresponding present capacity of the battery.
